# EUROPEAN PATENT APPLICATION

(11) **EP 2 136 202 A1**
(43) Date of publication of application: **23.12.2009**
(21) Application number: 08167679.3
(22) Date of filing: 27.10.2008
(51) Int. Cl.: G01N 22/04, G01N 33/38

(54) **Surface probe for moisture content measurement in building materials by time domain reflectometry comprising a transmission line made of two elastic conductors attached to an elastic dielectric made of foam to enable adaption to curved surfaces**

(30) Priority: 16.06.2008 PL 38544608
(71) Applicant: Politechnika Lubelska, 20-219 Lublin (PL)
(72) Inventor: Sobczuk, Henryk, 21-400 Swidnik (PL); Suchorab, Zbigniew, 21-010 Leczna (PL)
(74) Representative: Kaminski, Piotr

(57) **Abstract**

Probe for moisture content measurement especially in elements with curved surfaces is characterized in that it has elastic transmission line elements (2) which allow the probe to adjust to shape of the measured material or building object. Elements (2) are made from elastic metal and in section have the shape of flat bar allowing their deformation. The probe also has elastic dielectric (1) from isolating foam material, which allows for adjusting the elements (2) to conform with irregular shape of the measured surface.

## Description

The subject of the invention is a probe for moisture content measurement especially in elements with curved surfaces. The probe works with the application of time domain reflectometry method, and is utilized for moisture content measurement of porous building materials, building partitions, especially facades with curved surface, pillars and sculptures.

Known probe constructions are invasive in character because the principal measurement elements are metal rods of significant length of small diameter that during measurement have to be inserted through the holes into the material. This is the cause of montage problems especially in cases of hard materials measurement. In many cases this makes the measurement impossible or causes measurement errors.

This is the cause of montage problems especially in cases of hard materials measurement. In many cases this makes the measurement impossible or causes measurement errors.

Surface probe, according to Polish patent: PL 350713 allows for a non invasive measurement of moisture content of building partitions. Its characterized by rigid construction. The measuring elements are the metal angle bars which are placed on the flat surface of partition for the measurement. There is no need to insert the measuring elements into the structure of the partition. The described probe is a useful solution for humidity measurement in the flat structure elements.

The subject of the invention is the probe for moisture content measurement, especially of elements with curved surfaces, which has elastic measurement elements that allow the probe to adjust to the shape of measured material or building object. Measurement elements are made from elastic metal and in section they have a shape of a flat bar which allows for its deformation. The probe also has dielectric, elastic element made preferably from isolating foam material which allows the measurement elements to deform in order to conform with the irregular shape of the measured surface.

The advantage of the present invention is utilization of potential of time domain reflectometry method and its application for measurement of objects so far inaccessible. The probe for moisture content measurement, especially of elements of curved shape, can be applied for measurements of moisture content of hard building elements having irregular front and other architectural elements such as pillars, sculptures and piers. Measurements performed with use of probe according to this invention are possible irrespectively of the type and shape of measured detail.

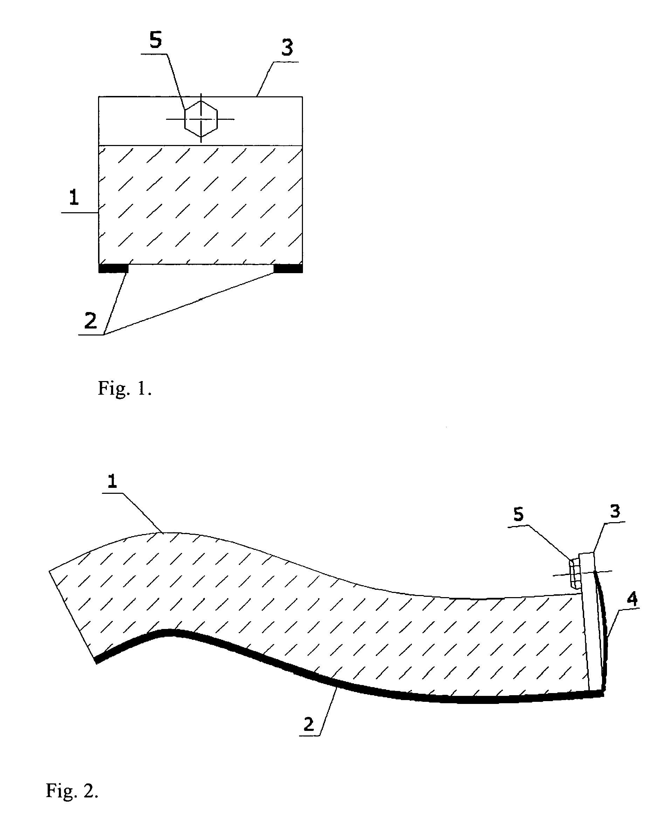

The embodiment of the invention is presented in the drawing, where Fig. 1 presents the probe in transversal cross section view while Fig.2 illustrates the probe in longitudinal cross section view.

The probe consists of two flat, elastic, placed in parallel measurement elements 2. Measurement elements 2 are separated with the elastic dielectric made from elastic material not susceptible to changes in humidity of the ambient air. The measurement elements 2 are connected by individual conductors 4 with connector 5 of the concentric cable connecting the probe with the readout device. In cross-section, the measuring elements 2 have a shape of flat bar and are made from elastic conducting material. Elastic dielectric element 1 keeps parallel measurement elements 2 in fixed distance. For construction stability the side plate 3 is provided. Along the plate, individual conductors are guided from the measurement elements to concentric cable connector 5, which is fixed still in the side plate 3.

For measurement, the probe for measurement of moisture content especially in curved surfaces, is placed on the measured surface and pushed against it so that the measurement elements 2 and elastic dielectric 1 deform in order to conform to the measured material on the entire length of the measurement elements 2. In this position, the measurement with the time domain reflectometry method is performed giving readout of the moisture content.

## Claims

1. Probe for moisture content measurement especially in elements with curved surfaces, **characterized in that** it has elastic measurement elements (2) which allow the probe to adjust to shape of the measured material or building object and measurement elements (2) are made from elastic metal and in section have the shape of flat bar allowing their deformation.

2. The probe according to claim 1, **characterized in that** it has elastic dielectric (1) from isolating foam material, which allows for adjusting the measurement elements (2) to conform with irregular shape of the measured surface.
